Europäisches Patentamt

European Patent Office

Office européen des brevets

(19)

(11) Publication number: **0 012 773**

**B1**

(12) **EUROPEAN PATENT SPECIFICATION**

(45) Date of publication of patent specification: **23.06.82**

(51) Int. Cl.³: **C 07 D 251/32**

(21) Application number: **79900082.3**

(22) Date of filing: **12.12.78**

(86) International application number:
**PCT/JP78/00050**

(87) International publication number:
**WO 79/00566 23.08.79 Gazette 79/17**

(54) Process for producing cyanuric acid.

(30) Priority: **31.01.78 JP 9755/78**

(43) Date of publication of application:
**09.07.80 Bulletin 80/14**

(45) Publication of the grant of the patent:
**23.06.82 Bulletin 82/25**

(84) Designated Contracting States:
**FR**

(56) References cited:
**JP - A - 50 062 987**
**JP - B - 35 003 373**
**JP - B - 41 004 715**
**JP - B - 49 025 676**
**US - A - 3 093 641**

(73) Proprietor: **NIPPON SODA CO., LTD.**
**2-1, Ohte-machi 2-chome**
**Chiyoda-ku, Tokyo, 100 (JP)**

(72) Inventor: **KIZAWA, Hidenori**
**2-33-15 Kamisoshigaya**
**Setagaya-ku, Tokyo 157 (JP)**
Inventor: **ICHIHARA, Ryoichi**
**1160-2, Ohaza Fujisawa Nakago-mura**
**Nakakubiki-gun, Niigata, 949-23 (JP)**
Inventor: **YAO, Toshio**
**5-24-2 Mukainohonmachi**
**Takaoka-shi, Toyama 933 (JP)**
Inventor: **KIKUCHI, Ichiro 2510-1, Simonagaya-**
**cho Koonan-ku**
**Yokohama-shi**
**Kanagawa, 233 (JP)**

(74) Representative: **Weinstein, Zinovi et al,**
**Cabinet Z. WEINSTEIN 20, Avenue de Friedland**
**F-75008 Paris (FR)**

Courier Press, Leamington Spa, England.

Process for producing cyanuric acid

This invention relates to a process for producing cyanuric acid from urea.

The commercial processes of producing cyanuric acid (hereinafter referred to as "CA") using urea as the starting raw material consist basically of heating urea at 180—300°C for several hours to convert it to CA with evolution of ammonia gas. However, the conversion of urea to CA in the commercial operation involves such difficulties as summarized below:

(a) The reaction mass solidifies in the course of conversion, and it turns to an inclusion containing urea, biuret, CA and others which requires additional heating to convert the remaining urea and biuret to CA, however, the additional heating is not carried out effectively because of poor heat conduction of the solidified reaction mass.

(b) an excessive heating of the solidified reaction mass necessary for converting the intermediates into CA causes decomposition of CA formed in the mass.

(c) the solidified reaction product adheres so strongly to the wall of the reactor that the removal of product from the reactor is difficult.

Numerous methods have been proposed to overcome these problems, and they include such troublesome processes as employing a pulverized mixture of urea and the recycled CA for the starting raw material to be heated, as shown for example in the Japanese published examined patent application No 49—25676 (1974).

Further, a two-step heating of urea to produce cyanuric acid is known from JP-A-5 062 987 consisting in heating melted urea to a temperature of 180° to 300°C.

It is therefore an object of this invention to provide a process of producing CA from urea, whereby it is possible to overcome difficulties existing in the conventional processes.

It is another object of this invention to provide a process of the type described, whereby the process is suitable for the commercial production of CA from urea.

In a process for producing cyanuric acid comprising the step of heating substantially pure melted urea or melted urea containing melted biuret to a temperature of 180° to 300°C, the present invention consists in heating said melted urea by means of a high frequency induction heating method.

The fused state urea used as the starting raw material of this invention is prepared by heating conventionally available solid urea at a temperature above its melting point of about 135°C. The fused state urea is not necessarily composed of substantially pure melted urea but it may contain an amount of melted biuret which is formed when urea is heated to obtain melted urea. Accordingly, the fused state urea is preferably prepared by heating urea at 140—150°C for obtaining the substantially pure melted urea, or by heating urea at 160—200°C for obtaining a melted state urea containing up to 30% of biuret.

When the fused state urea is placed in a high frequency electric field, it is susceptible of generating heat due to its dielectric loss, and the dielectric heat thus generated converts the urea or biuret to CA. Since the generation of dielectric heat occurs internally in molecules of melted urea or melted biuret, the heating method eliminates the heat transfer problem of the conventional methods employing the external heating of the reaction mass. Furthermore, the generation of dielectric heat occurs solely in melted urea and melted biuret, and does not occur in the product CA. That is to say, the heat generation continues so far as the raw material remains in the reaction mass, and after the entire raw material has been converted to solid CA, the heat generation ceases. Accordingly, the inclusion of the raw material in the solid CA, and the excessive heating of the product are minimized.

Since the chemical reaction according to this invention is the same as that of the conventional process, the temperature applied for heating of the reaction mass in this invention is 180—300°C. However, due to the efficient heating system of this invention, the duration of heating is reduced remarkably to 3—30 minutes, although it depends on the temperature of reaction mass.

The preferable heating conditions are composed of maintaining the reaction mass at 200—250°C for the duration of 5—20 minutes. Under these conditions, the reaction mass can be heated in vessels made of such electrically non-conductive materials as heat-resistant glass and heat-resistant plastics like glassfiber reinforced fluorocarbon resin, so that the product CA can be removed quite easily from the reaction vessel.

Apparatus employed for the high frequency induction heating are usually composed of electrodes and high frequency generators, and such factors as the shape, dimension and arrangement of the electrodes as well as the frequency and electrical power of the generators can be selected properly in accordance with the process conditions. The preferable frequencies of the generator are 10—3000 MHz, and the preferable shape of the electrode is parallel metal sheets.

When the fused state urea is set in the high frequency induction heating apparatus, and electrical power is supplied to maintain the reaction mass at the desired temperature, the reaction proceeds with evolution of ammonia gas. The power consumption for the heating continues until the reaction mass is converted entirely to solid CA, so that proceeding of the reaction may favourably be checked by the power consumption. The product CA thus obtained usually contains about 20—30% of impurities comprising mostly ammelide and ammeline. Therefore, the crude CA is treated with a 10% sulfuric acid to obtain purified CA.

According to the present invention, CA can be produced easily from urea in the yield of above 80%; so that the process is suitable for the commercial operation of CA production.

Example 1

Solid urea was heated at 150°C to prepare substantially pure melted urea. Into a 200 ml heat-resistant glass beaker was charged 100 g of the melted urea, and the beaker was inserted between parallel metal sheet electrodes connected with a high frequency generator for 27.12 MHz. By supplying electrical power to the generator, the content in the beaker began to be heated, and turned gradually to opaque with evolution of ammonia gas. Under controlling the power supply, the reaction mass was maintained at 200—250°C for about 10 minutes. The solid white product of 60.4 g thus obtained had the composition of CA 76.4%, ammelide 20.7% and others 2.9%. The crude CA was treated with a 10% sulfuric acid to obtain 58.2 g CA. The yield was 81.1%.

Example 2

One hundred grams of substantially pure melted urea in a 200 ml heat-resistant glass beaker was heated with a high frequency induction heater for 2450 MHz. After heating the content at 200—250°C for 8 minutes, 62.0 g of white solid was obtained. The crude CA had the composition of CA 78.3%, ammelide 20.4% and others 1.3%. By treating it with a 10% sulfuric acid was obtained 60.1 g of CA in 83.9% yield.

Example 3

Into a glassfiber reinforced fluorocarbon resin pan was charged 675 g of a fused state urea which contained about 7% of biuret formed during heating of urea at 170°C. The fused state urea in the pan was placed on parallel electrodes composing a grid electrode type high frequency induction heater for 13.56 MHz. After heating the content at 200—250°C for 15 minutes, 430 g of white solid was obtained. The crude CA had the composition of CA 66.6%, ammelide 31.8% and 1.6% others. The yield of purified CA was 79%.

Claims

1. A process for producing cyanuric acid comprising the step of heating substantially pure melted urea or melted urea containing melted biuret to a temperature of 180° to 300°C, characterized in that said heating is carried out by means of a high frequency induction heating method.

2. A process as claimed in claim 1 characterized in that said melted state urea contains melted biuret in an amount of up to 30%.

3. A process as claimed in claim 1 or 2 characterized in that said heating is carried out at from 200°C to 250°C.

4. A process as claimed in claims 1, 2 or 3 characterized in that said high frequency induction heating employs high frequency electric current of from 10 MHz to 3000 MHz.

**Revendications**

1. Un procédé de production d'acide cyanurique comprenant l'opération de chauffage d'urée fondue sensiblement pure ou d'urée fondue contenant du biuret jusqu'à une température de 180°C à 300°C, caractérisé en ce que ledit chauffage est exécuté au moyen d'une méthode de chauffage par induction à haute fréquence.

2. Un procédé tel que revendiqué dans la revendication 1, caractérisé en ce que l'urée précitée à l'état fondu contient du biuret fondu en une quantité allant jusqu'à 30%.

3. Un procédé tel que revendiqué dans la revendication 1 ou 2, caractérisé en ce que le chauffage précité est exécuté à une température de 200°C à 250°C.

4. Un procédé tel que revendiqué dans les revendications 1, 2 ou 3, caractérisé en ce que le chauffage précité par induction à haute fréquence emploie du courant électrique à haute fréquence de 10 MHz à 3000 MHz.

**Patentansprüche**

1. Ein Verfahren zur Herstellung von Zyanursäure mit dem Schritt der Erwärmung von im wesentlichen reinen geschmolzenen Harnstoff oder von geschmolzenen Biuret enthaltenden geschmolzenen Harnstoff bis zur einer Temperatur von 180°C bis 300°C, dadurch gekennzeichnet, dass die genannte Erwärmung mittels eines Hochfrequenzinduktionserhitzungverfahrens durchgeführt wird.

2. Ein Verfahren wie in Anspruch 1 beansprucht, dadurch gekennzeichnet, dass der genannte sich im geschmolzenen Zustand befindende Harnstoff geschmolzenen Biuret in einer Menge bis 30% enthält.

3. Ein Verfahren wie in Anspruch 1 oder 2 beansprucht, dadurch gekennzeichnet, dass die genannte Erwärmung bei einer Temperatur von 200°C bis 250°C durchgeführt wird.

4. Ein Verfahren wie in Ansprüche 1, 2 oder 3 beansprucht, dadurch gekennzeichnet, dass die genannte Hochfrequenzinduktionserwärmung einen elektrischen Hochfrequenzstrom von 10 MHz bis 3000 MHz verwendet.